(19) Europäisches Patentamt
European Patent Office
Office européen des brevets

(11) Veröffentlichungsnummer: **0 047 947**
**B1**

(12) # EUROPÄISCHE PATENTSCHRIFT

(45) Veröffentlichungstag der Patentschrift: **14.03.84**

(51) Int. Cl.³: **C 07 C 141/04,** C 07 C 59/21, C 07 C 51/58, C 25 B 3/02

(21) Anmeldenummer: **81106999.6**

(22) Anmeldetag: **07.09.81**

(54) **Omega-Fluorsulfato-perfluor-(2-methyl-alkan-3-one) sowie Verfahren zur Herstellung von Perfluorisopropylketocarbonsäurefluoriden über die erstgenannten Verbindungen.**

(30) Priorität: **12.09.80 DE 3034499**

(43) Veröffentlichungstag der Anmeldung: **24.03.82 Patentblatt 82/12**

(45) Bekanntmachung des Hinweises auf die Patenterteilung: **14.03.84 Patentblatt 84/11**

(84) Benannte Vertragsstaaten: **BE CH DE FR GB IT LI NL**

(56) Entgegenhaltungen:
**DE - B - 1 917 630**
**US - A - 3 254 107**

(73) Patentinhaber: **HOECHST AKTIENGESELLSCHAFT, Postfach 80 03 20, D-6230 Frankfurt am Main 80 (DE)**

(72) Erfinder: **Millauer, Hans, Dr., An den sieben Bäumen 21b, D-6236 Eschborn (DE)**
Erfinder: **Schwertfeger, Werner, Dr., Erlenstrasse 8, D-6306 Langgöns (DE)**
Erfinder: **Siegemund, Günter, Dr., Frankfurter Strasse 21, D-6238 Hofheim am Taunus (DE)**

**EP 0 047 947 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Omega-Fluorsulfato-perfluor-(2-methyl-alkan-3-one) sowie Verfahren zur Herstellung von Perfluorisopropylketocarbonsäurefluoriden über die erstgenannten Verbindungen

Perfluorisopropylketocarbonsäurefluoride sind Zwischenprodukte für die Herstellung perfluorierter Ketocarbonsäureester mit einem der Ketogruppe benachbarten Isopropylrest, die ihrerseits u. a. wertvolle Wärmeträgerflüssigkeiten und oberflächenaktive Mittel mit chemischer und thermischer Stabilität sind (vgl. z. B. JP-OS Sho-54-1 63 521).

Diese Perfluorisopropylketocarbonsäureester besitzen die Struktur II. Nach bekannten Verfahren werden sie hergestellt durch Alkoholyse von Perfluorisopropylketocarbonsäurefluoriden der Formel I (Zh. Org. Khim. 11, 1626 (1975)),

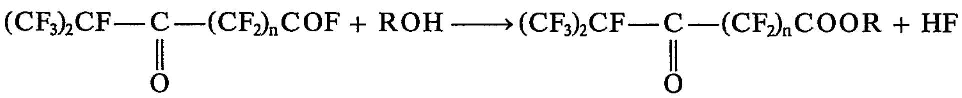

I II

wobei R = Alkyl und n = 2 – 5 bedeutet.

Ein anderes Verfahren, das aber nur auf n = 0 beschränkt ist, verwendet zur Herstellung Fluorglyoxalsäureester, die mit Hexafluorpropen umgesetzt werden (JP-OS Sho-54-163521):

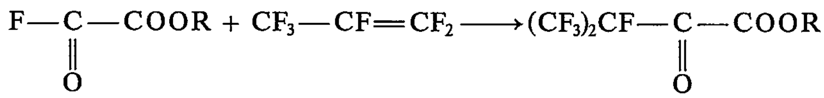

Neben der Beschränkung auf den Fall n = 0 hat dieses Verfahren den Nachteil, daß die Fluorglyoxalsäureester nur mit unbefriedigenden Ausbeuten erhalten werden. Zum Beispiel entsteht nach DE-OS 27 51 050 der Fluorglyoxalsäuremethylester mit einer Ausbeute von nur ca. 10%.

Andererseits sind Perfluorisopropylketocarbonsäurefluoride der Formel I mit n = 2 – 5 durch Umsetzung von Perfluordicarbonsäuredifluoriden III mit Hexafluorpropen hergestellt worden [Zh. Org. Khim. 11, 1626 (1975)],

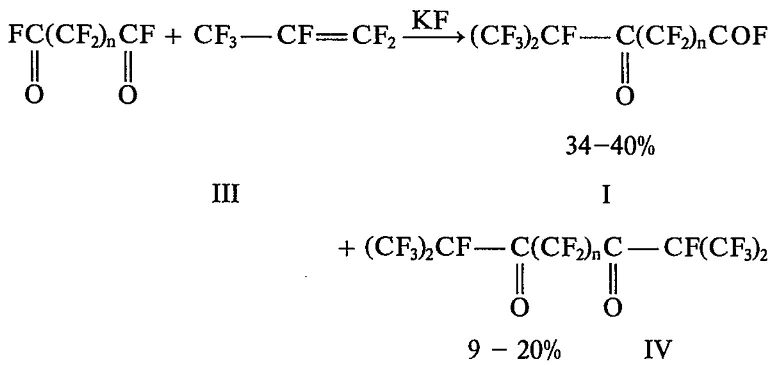


III I IV

wobei n = 2 – 5 bedeutet. Abgesehen davon, daß die Diketone der Formel IV, die bis zu 20% entstehen können, die Ausbeute an den gewünschten Produkten der Formel I beeinträchtigen und auch durch eine Nachreaktion nicht in diese übergeführt werden können, werden die als Vorprodukte verwendeten Dicarbonsäurefluoride der Formel III nur in sehr geringer Ausbeute erhalten.

Nach dem in der DE-OS 26 35 312 beschriebenen Verfahren werden solche perfluorierten Dicarbonsäuredifluoride aus Perfluoralkandiiodiden mit rauchender Schwefelsäure erhalten. Beschrieben ist die Herstellung des Perfluordicarbonsäuredifluorids III mit n = 2 aus Perfluorbutyldijodid:

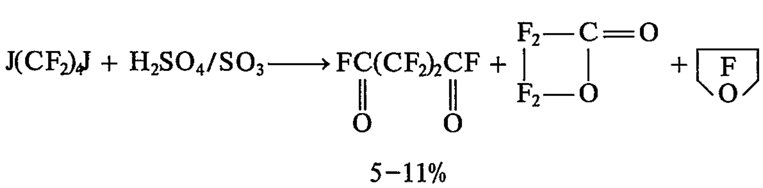


wobei neben 5–11% der gewünschten Verbindung hauptsächlich Perfluor-γ-butyrolacton und Perfluortetrahydrofuran gebildet werden.

Das Verfahren der japanischen Autoren I. Watanabe et al., ref. in C. A. Vol 85, S. 591 Nr. 85:93867 f (1976) geht von Perfluorcycloalkenen aus, die mit Sauerstoff/Ozon in die gewünschten Verbindungen III mit n = 2 – 4 überführt werden. Auch in diesem Fall sind die Ausbeuten unbefriedigend, wie das Beispiel für n = 2 zeigt:

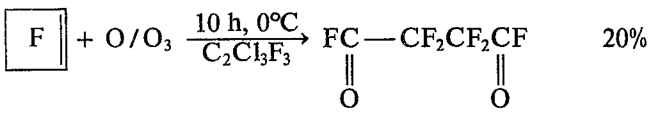


III mit n = 2

Wegen der großen Bedeutung der Perfluorisopropylketocarbonsäurefluoride als Vorprodukte für die technisch wichtigen Perfluorisopropylketocarbonsäureester und der für technische Realisierbarkeit unbefriedigenden Verfahren zu ihrer Herstellung bestand nun die Aufgabe, einen verbesserten Weg zu den Perfluorisopropylketocarbonsäurefluoriden zu erschließen, der mit höheren Ausbeuten und einheitlich zu den gewünschten Produkten führt.

Diese Aufgabe konnte erfindungsgemäß durch die Bereitstellung neuer Perfluorverbindungen, nämlich von $\omega$-Fluorsulfatoperfluor-(2-methyl-alkan-3-onen) der Formel V gelöst werden:

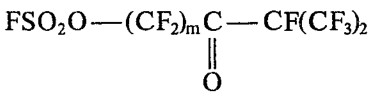

(V)

worin m = 1—10, vorzugsweise 1—8, insbesondere 1—6 bedeutet.

Zu den Perfluorisopropylketocarbonsäurefluoriden der Formel I kommt man — ausgehend von V — durch Zersetzung in Gegenwart von katalytischen Mengen Alkalifluorid und in Abwesenheit von aprotischen polaren Lösungsmitteln. Aus den Verbindungen V entstehen so in hohen Ausbeuten die perfluorierten Ketocarbonsäurefluoride der Formel I:

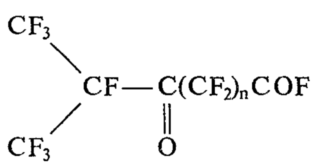

I

worin n = 0–9, vorzugsweise 0–7, insbesondere 0–5 bedeutet.

Die Verbindungen der Formel I werden erfindungsgemäß dadurch hergestellt, daß man

a) $\omega$-Hydro-perfluor-(2-methyl-alkan-3-one) der Formel VI

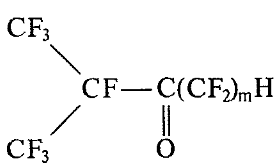

VI

worin m dieselbe Bedeutung wie in Formel V besitzt, in einem Elektrolyten aus Fluorsulfonsäure und einem Alkalifluorsulfonat unter Verwendung von Anoden aus Metallen der Platin-Gruppe (Osmium, Iridium, Platin) und/oder glasartigem Kohlenstoff, und von Kathoden aus einem üblichen, jedoch unter den Elektrolysebedingungen stabilen Material, elektrolysiert, die dabei gebildeten $\omega$-Fluorsulfato-perfluor(2-methyl-alkan-3-one) der Formel V

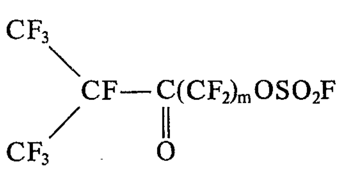

V

worin m die obengenannte Bedeutung besitzt, isoliert und

b) in Gegenwart katalytischer Mengen Alkalifluorid MF, wobei M = Li, Na, K, Rb und Cs sein kann, und in Abwesenheit von polaren aprotischen Lösungsmitteln zu den Perfluorisopropylketocarbonsäurefluoriden der Formel I zersetzt.

Obwohl perfluorierte Fluorsulfatoverbindungen schon durch anodische Oxidation hergestellt worden sind, z. B. $\omega$-Fluorsulfatoperfluoralkane in einer Mischung von Fluorsulfonsäure und einem Alkalifluorsulfonat unter Verwendung von Platinelektroden (ICS Chem. Comm. **1978**, 118), ist es für das praktisch komplikationslose Gelingen der Verfahrensstufe a überraschend, daß die Ketofunktion der $\omega$-Hydroperfluor(2-methyl-alkan-3-one) der Formel VI bei der Elektrolyse nicht angegriffen und somit nicht verändert wird.

Ebenso überraschend war die glatte Zersetzung der erfindungsgemäßen Verbindungen der Formel V in der Verfahrensstufe b in Gegenwart katalytischer Mengen Alkalifluorid zu den Perfluorisopropylketocarbonsäurefluoriden der Formel I, da nach Izv. Akad. Nauk SSSR, Ser. Khim. 1979, 3, 667—9 bekannt ist, daß in Gegenwart von Ketogruppen der Fluorsulfatorest nicht abgespalten wird, sondern eine Umlagerung stattfindet.

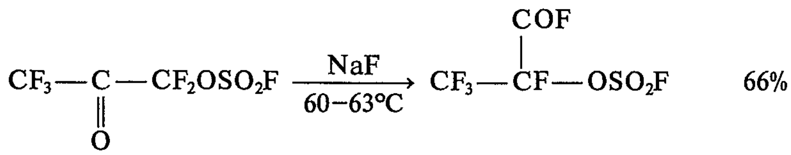


Die Ausgangsverbindungen für das erfindungsgemäße Verfahren — also die ω-H-perfluor-(2methylalkan-3-one) der Formel VI — können nach folgender bekannter Verfahrensweise durch Umsetzung von ω-Hydroperfluorcarbonsäurefluoriden VII mit Hexafluorpropen in Gegenwart eines Alkalimetallfluorids und Acetonitril hergestellt werden:

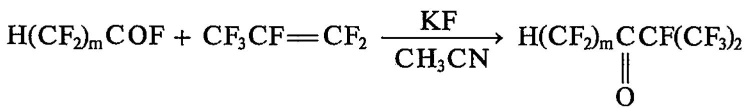

VI

VII

[I. P. Kolenko et al., ref. in C. A. 82, 97627 p (1975)]. Die ω-Hydroperfluorcarbonsäurefluoride lassen sich ihrerseits in literaturbekannter Weise herstellen:

1. J. Am. Chem. Soc. 74, 1426 (1952):
   Aus Ammoniak und Tetrafluorethylen wird in Gegenwart von Kupferacetat das nachstehende Triazinderivat hergestellt:

   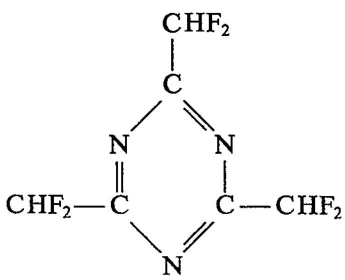

   aus dem durch Erhitzen mit wäßriger Natronlauge das Natriumdifluoracetat und daraus nach bekannten Methoden die Säurefluoride der Formel VII mit m = 1 erhältlich sind.
2. US-PS 2 559 629 beschreibt die Herstellung von aliphatischen Polyfluorcarbonsäuren und ihren Salzen durch Oxidation von Polyfluoralkanolen mit Kaliumpermanganat:

   $$H(CX_2CX_2)_nCH_2OH \xrightarrow{\text{Oxidation}} H(CX_2CX_2)_nCOOH$$

   X = Cl, F, mit mindestens der Hälfte der Reste X = F, n = 1—3
   Die Ausgangsverbindungen für diese Oxidation werden aus Ethylenderivaten $CX_2 = CX_2$ und Methanol hergestellt.
   Aus den erhaltenen freien Säuren werden die Säurefluoride auf bekanntem Wege gewonnen; die Verbindungen mit den Resten X = F stellen die Verbindungen der Formel VII mit m = gerade Zahlen dar.
3. J. Org. Chem. 42, 25, 4055 (1977) beschreibt u. a. folgende Reaktion:

   $$H(CF_2)_6CH_2OH \xrightarrow{\text{Oxidation}} H(CF_2)_6COOH \xrightarrow[\text{Rückfluß}]{C_6H_5—COCl} H(CF_2)_6COCl$$

   $$H(CF_2)_6COCl \xrightarrow[\text{Diglyme}]{\text{NaF}} H(CF_2)_6COF$$

Die Verfahrensstufe a — die Elektrolyse des erfindungsgemäßen Verfahrens — wird im Prinzip in der etwa aus J. C. S., chem. Comm. 1978, 118 bekannten Weise durchgeführt.

Zur Bereitung des Grundelektrolyten, welcher erfindungsgemäß aus Fluorsulfonsäure und einem darin gelösten Alkalimetallfluorsulfonat besteht, löst man ein entsprechendes, leicht zugängliches Alkalimetallchlorid, wie z. B. Lithiumchlorid, Natriumchlorid oder Kaliumchlorid in Fluorsulfonsäure,

die man gegebenenfalls vorher einer Reinigung durch fraktionierte Destillation unterzogen hat, wobei sofort die Hauptmenge an Chlorwasserstoff aus der Lösung entweicht. Der Rest wird durch Einleiten von trockenem Stickstoff ausgetrieben. Die anzuwendende Konzentration des Alkalimetallsulfonats im Grundelektrolyten ist nicht kritisch und liegt im Bereich von etwa 0,05 bis 3 Mol pro Liter.

Die Ausgangsstoffe der Formel VI werden im Grundelektrolyten gelöst oder dispergiert, wobei Gemische mit bis zu etwa 60%, bezogen auf den Grundelektrolyten, zur Anwendung kommen können.

Als Anode eignen sich Osmium, Iridium, Platin oder Platinlegierungen mit bis zu etwa 10% an anderen Edelmetallen, beispielsweise Iridium. Vorzugsweise besteht die Anode jedoch aus glasartigem Kohlenstoff (Glassy Carbon), der sich unter den Elektrolysebedingungen als besonders korrosionsbeständig erwiesen hat. Glasartiger Kohlenstoff eignet sich auch als Kathode, obwohl die Materialfrage für diese Elektrode nicht kritisch ist und andere Stoffe wie beispielsweise Platin, Kupfer oder Edelstahl dafür ebenfalls geeignet sind. Über die Herstellung, Struktur und Eigenschaften von glasartigem Kohlenstoff siehe z. B. den Artikel ›Thermischer Abbau von Polymeren bis zum elementaren Kohlenstoff — ein Weg zu Werkstoffen der Zukunft‹ von E. Fitzer in Angew. chem. 92, 375—386 (1980).

Das Flächenverhältnis von Anode zu Kathode liegt zwischen etwa 1 : 1 und etwa 10 : 1, vorzugsweise zwischen etwa 5 : 1 und etwa 10 : 1.

Die Elektrolyse wird bei einer anodischen Stromdichte von etwa 10—150 $mA \cdot cm^{-2}$, vorzugsweise von etwa 20—80 $mA \cdot cm^{-2}$ und einer Temperatur von etwa 0 bis 100°C, vorzugsweise etwa 20 bis 40°C betrieben.

Die Aufarbeitung der Elektrolysegemische und die Isolierung der ω-Fluorsulfato-perfluor(2-methylalkan-3-one) der allgemeinen Formel V erfolgt in an sich bekannter Weise. Bei zweiphasigen Reaktionsgemischen ist es zweckmäßig, die fluororganische Phase, die gegebenenfalls nur noch wenig Fluorsulfonsäure enthält, durch Scheiden abzutrennen; anderenfalls muß das Elektrolyseprodukt von dem Grundelektrolyten destillativ abgetrennt werden. In beiden Fällen kann die Elektrolytphase bzw. der Destillationssumpf nach Zugabe frischer Fluorsulfonsäure wieder in die Elektrolysestufe recyclisiert werden.

Die rohen ω-Fluorsulfato-perfluor(2-methyl-alkan-3-one) V können durch fraktionierte Destillation weiter gereinigt werden. Unter Umständen ist es zweckmäßig, die Rohprodukte durch vorheriges Behandeln mit Natriumfluorid oder Auswaschen mit Natriumbicarbonat-Lösung zu entsäuern.

Die zweite Stufe b) des erfindungsgemäßen Verfahrens besteht in der Umsetzung der ω-Fluorsulfato-perfluor(2-methyl-alkan-3-one) der allgemeinen Formel V in Gegenwart eines geeigneten, nucleophilen Katalysators zu den ω-Fluorcarbonyl-perfluor(2-methyl-alkan-3-onen) der allgemeinen Formel I.

Katalysatoren für das erfindungsgemäße Verfahren sind die Alkalifluoride (LiF, NaF, KF, RbF, CsF). Sie können sowohl einzeln als auch in Mischung miteinander eingesetzt werden. Die Katalysatormenge liegt im allgemeinen zwischen etwa 1 und 50 Mol %, vorzugsweise zwischen etwa 10 und 30 Mol %, bezogen auf die Fluorsulfato-Verbindung der Formel V.

Die Reaktionstemperaturen liegen je nach verwendetem Katalysator im allgemeinen in dem Bereich zwischen etwa —20 und +120°C, vorzugsweise zwischen etwa 0 und +100°C.

Es kann sowohl bei Normaldruck als auch unter Überdruck gearbeitet werden.

Ohne Belang ist es für die Umwandlung der ω-Fluorsulfato-perfluor-(2-methyl-alkan-3-one) der Formel V in die Perfluorisopropylketocarbonsäurefluoride der Formel I auf die erfindungsgemäße Weise, in welcher Reihenfolge die Reaktionspartner und das gegebenenfalls verwendete Lösungsmittel vereinigt werden. Von Vorteil ist es jedoch, für eine gute Durchmischung des Ansatzes während der gesamten Reaktionsdauer zu sorgen.

Nach einer bevorzugten Arbeitsweise wird der Katalysator vorgelegt, das ω-Fluorsulfato-perfluor-(2-methyl-alkan-3-on) der Formel V bei Raumtemperatur zugegeben und das Reaktionsgemisch bis zum Auftreten einer Gasentwicklung langsam aufgeheizt. Nachdem die Gasentwicklung beendet ist, wird das Perfluorisopropylketocarbonsäurefluorid der Formel I unter Verwendung einer Kolonne destillativ vom Katalysator abgetrennt.

Nachstehende Beispiele sollen die Erfindung näher erläutern.

Danach folgt dann noch ein Vergleich der Herstellung eines beispielhaften Perfluorisopropylcarbonsäurefluorids — nämlich der Verbindung

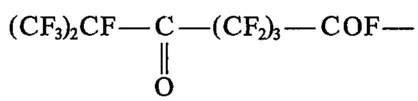

ausgehend von einfachen Basisprodukten, über die erfindungsgemäße entsprechende ω-Fluorsulfatoverbindung und nach dem Stand der Technik. Der durch die Erfindung ermöglichte Weg ergibt eine etwas bessere Gesamtausbeute bei zum Teil einfacheren Verfahrensstufen.

## Beispiel 1

Darstellung von 4-Fluorsulfato-perfluor(2-methyl-butan-3-on).

$$\left((CF_3)_2CF-\underset{\underset{O}{\|}}{C}-CF_2-OSO_2F\right)$$

Die Elektrolysevorrichtung besteht aus einem mit einem äußeren Kühlmantel und einem Deckel versehenen zylindrischen Glasgefäß von etwa 60 mm Innendurchmesser und 100 mm Höhe. Die Zelle ist mit einem als Rückflußkühler wirkenden Trockeneiskühler, ferner mit einem Gaseinleitungsrohr, Thermometer und den Stromzuführungen für die Elektroden versehen. Im Abstand von etwa 20 mm vom Boden befindet sich eine zylinderförmige Anode aus Platinnetz (Durchmesser 40 mm; Höhe 40 mm; Maschenabstand ca. 1 mm), die an der Unterseite des Zelldeckels befestigt ist. Ein zweiter Zylinder aus Platinnetz (Durchmesser 12 mm; Höhe 40 mm) ist ebenfalls am Deckel der Zelle gehaltert und dient als Kathode. Als Rührer wird ein PTFE-umhüllter Magnetstab auf dem Boden der Zelle benutzt. Die Kühlung der Zelle erfolgt durch einen externen Kühlkreislauf mit Perchlorethylen als Kühlflüssigkeit.

Alle medienberührten Teile der Vorrichtung bestehen aus Glas, Platin oder PTFE.

Der Grundelektrolyt wird durch Zugabe von 12,5 g (0,16 Mol) Kaliumchlorid zu 250 g destillierter Fluorsulfonsäure hergestellt; es bildet sich eine farblose Lösung, die durch Einleiten von trockenem Stickstoff von restlichem Chlorwasserstoff befreit und anschließend 4 Stunden bei einer Stromstärke von 2 A vorelektrolysiert wird. Nach Zugabe von 85,5 g (0,34 Mol) 4-Hydro-perfluor(2-methyl-butan-3-on) wird bei einer Stromstärke von 2 A und einer Temperatur von 25°C elektrolysiert, bis ein Ladungsdurchgang von 57 AH erreicht ist. Die Zellspannung beträgt 5—6 V.

Nach Beendigung der Elektrolyse wird das Elektrolysegemisch bis zu einer Sumpftemperatur von 165°C andestilliert und das Destillat (125 g) durch eine weitere fraktionierte Destillation aufgetrennt. Man erhält als Fraktion 27,3 g (35% d. Th. bezogen auf umgesetztes Ausgangsprodukt) 4-Fluorcarbonyl-perfluor(2-methyl-butan-3-on, Kp. 87—88°C.

$$^{19}F—NMR(CDCl_3)^{**}: +50{,}8\ (1F, —OSO_2F);\ -74{,}0\ (6F, —CF_3);$$
$$-78{,}0\ (2F, —CF_2—);\ -191{,}5\ (1F, >CF)$$

** Für alle $^{19}F$-NMR-Spektren dient $CFCl_3$ als innerer Standard.

## Beispiel 2

Darstellung von 3-Fluorcarbonyl-perfluor(2-methyl-propan-3-on)

$$\left((CF_3)_2CF-\underset{\underset{O}{\|}}{C}-COF\right)$$

In einem ausgeheizten 100 ml Dreihalskolben mit Magnetrührer, Thermometer, Tropftrichter und 20 cm Raschig-Ring-Kolonne mit Destillationskopf und Kältekühler werden 2,5 g (0,016 Mol) trockenes Cäsiumfluorid vorgelegt. Bei Raumtemperatur werden anschließend 30 g (0,08 Mol) 4-Fluorsulfato-perfluor(2-methyl-butan-3-on) zugegeben. Dabei entwickelt sich Sulfurylfluorid. Nach 3stündigem Rühren bei 22°C wird das Produkt destillativ vom Cäsiumfluorid abgetrennt. Es werden 11 g (0,045 Mol) 3-Fluorcarbonyl-perfluor(2-methyl-propan-3-on), Siedepunkt 32—33°C bei 753 mm, erhalten, entsprechend einer Ausbeute von 56% der Theorie.

$C_5F_8O_2$ (Molekulargewicht: 244) Ber.: C 24,6% F 62,2%
Gef.: C 23,9% F 59,8%

$$^{19}F—NMR(CDCl_3): +27{,}2\ (1F, —COF);\ -73{,}3\ (6F, —CF_3);$$
$$-192{,}7\ (1F, >CF)$$

## Beispiel 3

Darstellung von 3-Fluorsulfato-perfluor(2-methyl-pentan-3-on)

$$\left((CF_3)_2CF-\underset{\underset{O}{\|}}{C}-CF_2CF_2-OSO_2F\right)$$

Unter Verwendung einer Elektrolysevorrichtung und nach Zubereitung eines Grundelektrolyten aus 250 g Fluorsulfonsäure und 12,5 g (0,16 Mol) Kaliumchlorid, wie in Beispiel 1 beschrieben, werden 99 g (0,33 Mol) 5-Hydro-perfluor(2-methyl-pentan-3-on) bei einer Stromdichte von 2 A und einer Zellspannung von 6—11 V elektrolysiert. Die Elektrolysetemperatur beträgt 25°C. Nach einem Ladungsdurchgang von 63 Ah wird die Elektrolyse beendet und das Reaktionsgemisch im Scheidetrichter getrennt. Die fluororganische Phase (145 g) wird an einer 30 cm Füllkörperkolonne fraktioniert destilliert. Man erhält als Fraktion 84 g (84% der Theorie, bezogen auf umgesetztes Ausgangsmaterial) 5-Fluorsulfato-perfluor(2-methyl-pentan-3-on), Siedepunkt 104—105°C.

$$^{19}F-NMR(CDCl_3):\ +51{,}7\ (1F, -OSO_2F);\ -73{,}0\ (6F, -CF_3);$$
$$-83{,}4\ (2F, -OCF_2-);\ -119{,}0\ (2F, -CO-CF_2-);$$
$$-189{,}4\ (1F, >CF)$$

## Beispiel 4

Darstellung von 4-Fluorcarbonyl-perfluor(2-methyl-butan-3-on)

$$\left((CF_3)_2CF-\underset{\underset{O}{\|}}{C}-CF_2COF\right)$$

In einem ausgeheizten 250 ml Dreihalskolben mit Magnetrührer, Tropftrichter, Thermometer und Kühler (—20°C) mit angeschlossener Falle (—78°C) werden bei Raumtemperatur 5 g (0,033 Mol) trockenes Cäsiumfluorid vorgelegt und 92 g (0,23 Mol) 5-Fluorsulfato-perfluor(2-methyl-pentan-3-on) zugegeben, wobei sich Sulfurylfluorid entwickelt und die Innentemperatur von 24 auf 30°C ansteigt. Nach Aufheizen auf 60°C nimmt die Gasentwicklung zu. Innerhalb von 20 Minuten hört sie jedoch auf, es bildet sich Rückfluß. Bei der folgenden Destillation über eine 50 cm Raschig-Ring-Kolonne werden 51 g (0,173 Mol) 4-Fluorcarbonyl-perfluor(2-methyl-butan-3-on) mit einem Siedepunkt von 58°C bei 766 mm erhalten. (Ausbeute: 75,6 % der Theorie)

$C_6F_{10}O_2$ (Molekulargewicht: 294) Ber.: C 24,5% F 64,6%
Gef.: C 24,4% F 63,6%

$$^{19}F-NMR(CDCl_3):\ +24{,}3\ (1F, -COF);\ -73{,}2\ (6F, -CF_3);$$
$$-111{,}0\ (2F, -CF_2-);\ -190{,}8\ (1F, >CF)$$

## Beispiel 5

Darstellung von 7-Fluorsulfato-perfluor(2-methyl-heptan-3-on)

$$\left((CF_3)_2CF-\underset{\underset{O}{\|}}{C}-(CF_2)_4-OSO_2F\right)$$

Es wird eine Elektrolysevorrichtung, wie in Beispiel 1 beschrieben, benutzt jedoch als Anode eine Platte (100 × 20 × 3 mm) aus glasartigem Kohlenstoff sowie als Kathode ein Stab (Durchmesser 3 mm, Länge 100 mm) aus demselben Material verwendet. Nach Bereitung eines Grundelektrolyten aus 250 g Fluorsulfonsäure und 10,6 g (0,25 Mol) Lithiumchlorid in analoger Weise wie in Beispiel 1 beschrieben, werden 185 g (0,46 Mol) 7-Hydro-perfluor(2-methyl-heptan-3-on) 15 Stunden bei einer Stromdichte

von 2 A und einer Zellspannung von 13—15 V elektrolysiert. Die Elektrolysetemperatur beträgt 20°C. Anschließend wird das Elektrolysegemisch im Scheidetrichter getrennt und die fluororganische Phase (194 g) an einer 30 cm Füllkörperkolonne fraktioniert destilliert. Man erhält als Fraktion 124 g (66% der Theorie, bezogen auf umgesetztes Ausgangsprodukt) 7-Fluorsulfato-perfluor(2-methyl-heptan-3-on), Siedepunkt 143—144°C.

$^{19}F$—NMR($CDCl_3$): + 51,5 (1 F, —$OSO_2F$); − 73,2 (6 F, —$CF_3$); − 82,8 (2 F, —O—$CF_2$—); − 116,4 (2 F, —$CF_2$—); − 120,7 (2 F, —$CF_2$—); − 123,3 (2 F, —$CF_2$—); − 190,2 (1 F, $>$CF)

Beispiel 6

Darstellung von 6-Fluorcarbonyl-perfluor(2-methyl-hexan-3-on)

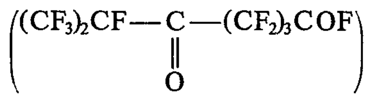

In einem 200 ml Dreihalskolben mit Magnetrührer, 20 cm Raschig-Ring-Kolonne und Kältedestillationskopf, Tropftrichter sowie Thermometer werden bei Raumtemperatur 8 g (0,053 Mol) trockenes Cäsiumfluorid und 223 g (0,45 Mol) 7-Fluorsulfato-perfluor(2-methyl-heptan-3-on) vereinigt und auf 75°C erwärmt. Dabei entwickelt sich Sulfurylfluorid, das in einer Falle kondensiert wird. (43 g = 0,42 Mol = 93% der Theorie). Bei der fraktionierten Destillation wird eine Fraktion von 160 g (0,41 Mol) 6-Fluorcarbonyl-perfluor(2-methyl-hexan-3-on) mit einem Siedepunkt von 102°C erhalten, was einer Ausbeute von 90% der Theorie entspricht.

$C_8F_{14}O_2$ (Molekulargewicht: 394) Ber.: C 24,36% F 67,51%
Gef.: C 24,1% F 67,5%

$^{19}F$—NMR($CDCl_3$): + 24,63 (1 F, —COF); − 74,01 (6 F, —$CF_3$); − 116,8 (2 F, —$CF_2$—); − 118 (2 F, —$CF_2$—); − 122,3 (2 F, —$CF_2$—); − 191 (1 F, $>$CF)

Vergleich: Herstellung von

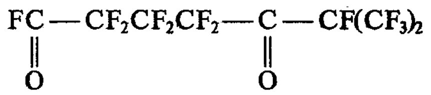

1. Über die erfindungsgemäße ω-Fluorsulfatoverbindung

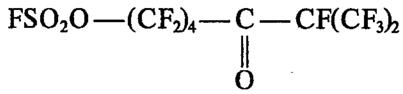

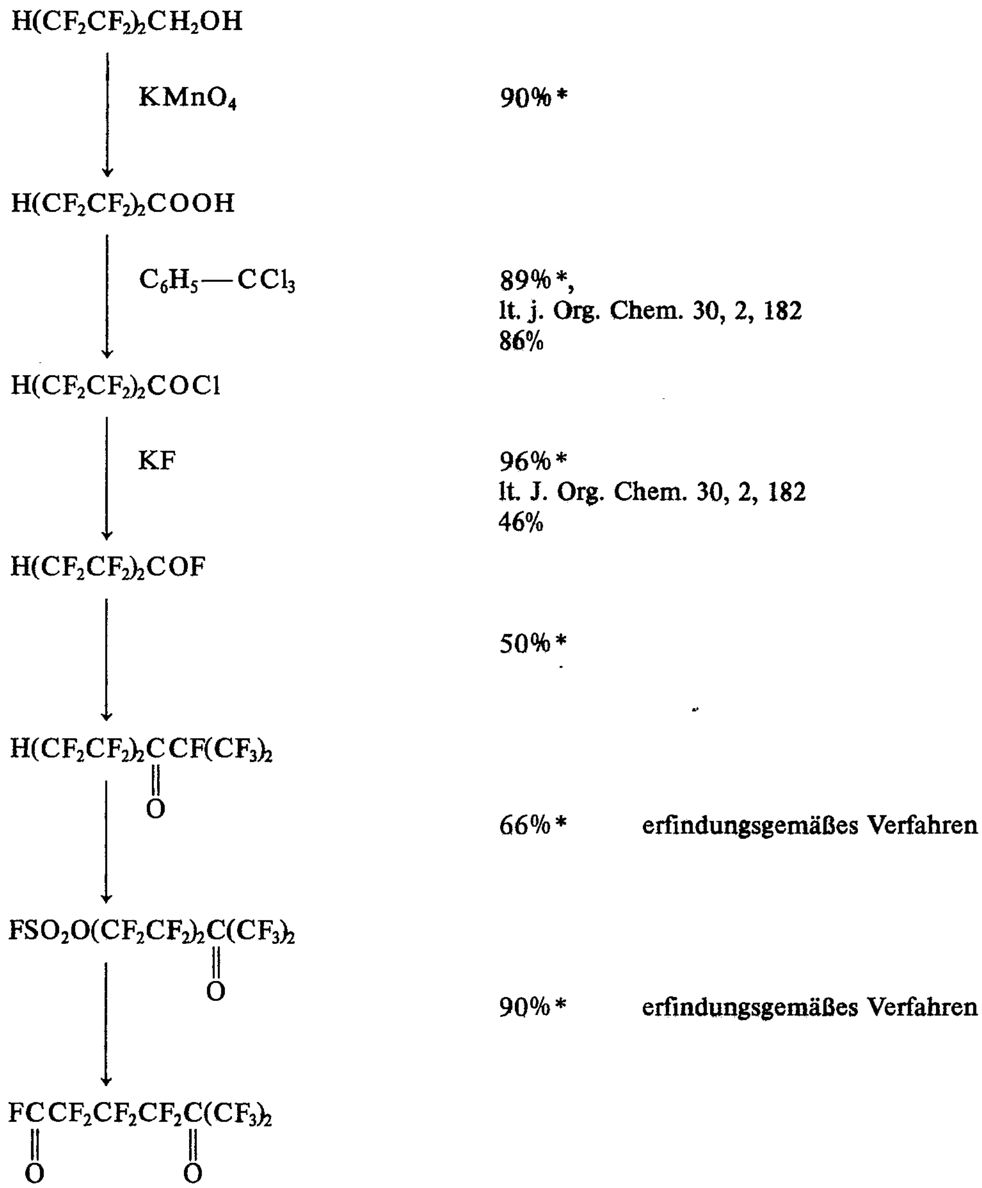


* = in eigenen Versuchen erreichte Ausbeuten.

Ausbeute bezogen auf $H(CF_2CF_2)_2CH_2OH$ : 22,8%.

2. Nach dem Stand der Technik

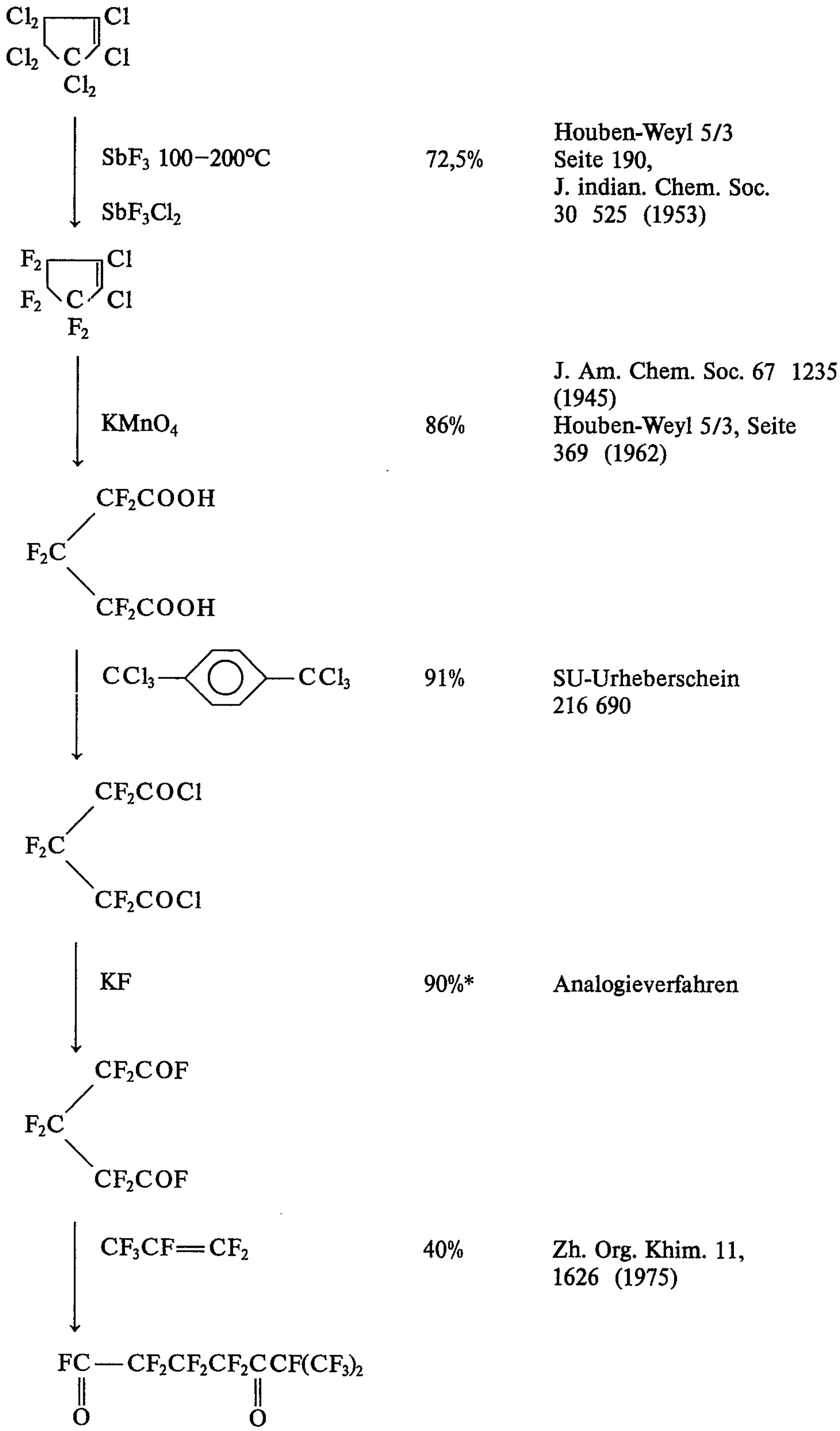


Gesamtausbeute bezogen auf Perchlorcyclopenten: 20,4%.

**Patentansprüche:**

1. ω-Fluorsulfato-perfluor-(2-methyl-alkan-3-one) der Formel V:

$$FSO_2O-(CF_2)_m-\underset{\displaystyle O}{\underset{\|}{C}}-CF(CF_3)_2 \qquad (V)$$

worin m = 1–10, vorzugsweise 1–8, insbesondere 1–6.

2. Verfahren zur Herstellung von Perfluorisopropylketocarbonsäurefluoriden der Formel I

$$FOC-(CF_2)_n-\underset{\displaystyle O}{\underset{\|}{C}}-CF(CF_3)_2 \qquad (I)$$

worin n = 0–9, vorzugsweise 0–7, insbesondere 0–5, und wobei n = m – 1 gilt (Bedeutung von m siehe unten), dadurch gekennzeichnet, daß man

a) ω-Hydro-perfluor-(2-methyl-alkan-3-one) der Formel VI

$$H-(CF_2)_m-\underset{\displaystyle O}{\underset{\|}{C}}-CF(CF_3)_2 \qquad (VI)$$

worin m = 1–10, vorzugsweise 1–8, insbesondere 1–6, in einem Elektrolyten aus Fluorsulfonsäure und einem Alkalifluorsulfonat unter Verwendung von Anoden aus Metallen der Platingruppe (Os, Ir, Pt) und/oder glasartigem Kohlenstoff und von Kathoden aus einem üblichen, jedoch unter den Elektrolysebedingungen stabilen Material elektrolysiert, die dabei gebildeten ω-Fluorsulfato-(2-methyl-alkan-3-one) der Formel V

$$FSO_2O-(CF_2)_m-\underset{\displaystyle O}{\underset{\|}{C}}-CF(CF_3)_2 \qquad (V)$$

worin m die obige Bedeutung besitzt, isoliert und

b) in Gegenwart katalytischer Mengen Alkalifluorid MF (M=Li, K, Na, Rb, Cs) und in Abwesenheit von polaren aprotischen Lösungsmitteln zu den Perfluorisopropylketocarbonsäurefluoriden der Formel I zersetzt.

**Claims:**

1. ω-fluorosulfato-perfluoro-(2-methyl-alkan-3-ones) of the formula V:

$$FSO_2O-(CF_2)_m-\underset{\displaystyle O}{\underset{\|}{C}}-CF(CF_3)_2 \qquad (V)$$

in which m = 1–10, preferably 1–8 and in particular 1–6.

2. A process for the preparation of perfluoroisopropylketocarboxylic acid fluorides of the formula I

$$FOC-(CF_2)_n-\underset{\displaystyle O}{\underset{\|}{C}}-CF(CF_3)_2 \qquad (I)$$

in which n = 0–9, preferably 0–7 and in particular 0–5, and whereby n = m – 1 (meaning of m see below) characterized in

a) electrolyzing ω-hydro-perfluoro-(2-methyl-alkan-3-ones) of the formula VI

$$H-(CF_2)_m-\underset{\displaystyle O}{\underset{\|}{C}}-CF(CF_3)_2 \qquad (VI)$$

in which m = 1–10, preferably 1–8 and in particular 1–6, in an electrolyte consisting of fluorosulfonic acid and an alkali metal fluorosulfonate, using anodes made of metals of the platinum group (Os, Ir, Pt) and/or glassy carbon, and cathodes made of a material which is customary, but stable under the electrolysis conditions, isolating the ω-fluorosulfato-(2-methyl-alkan-3-ones) thereby formed, of the formula V

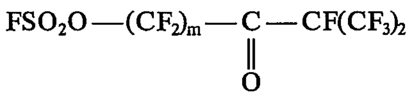
(V)

in which m has the above meaning, and

b) decomposing these compounds in the presence of catalytic amounts of an alkali metal fluoride MF (M = Li, K, Na, Rb or Cs) and in the absence of polar aprotic solvents to give the perfluoroisopropylketocarboxylic acid fluorides of the formula I.

**Revendications**

1. ω-Fluorosulfato-perfluoro-(méthyl-2 alcanones-3) répondant à la formule V

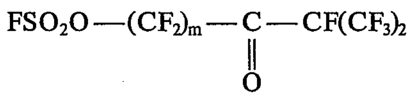
(V)

dans laquelle m est un nombre de 1 à 10, de préférence de 1 à 8 ou, mieux encore, de 1 à 6.

2. Procédé de préparation de fluorures d'acides perfluoroisopropyl-céto-carboxyliques répondant à la formule I:

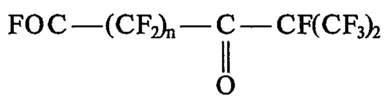
(I)

dans laquelle n est un nombre de 0 à 9, de préférence de 0 à 7 ou, mieux encore, de 0 à 5, et n est égal à m − 1 (m ayant la signification indiquée ci-dessous), procédé caractérisé en ce que:

a) on électrolyse des ω-hydro-perfluoro-(méthyl-2 alcanones-3) répondant à la formule VI:

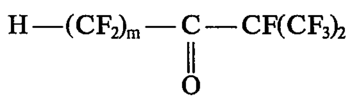
(VI)

dans laquelle m est un nombre de 1 à 10, de préférence de 1 à 8 ou, mieux encore, de 1 à 6, dans un électrolyte constitué d'acide fluorosulfonique et d'un fluorosulfonate de métal alcalin, en utilisant des anodes en métaux de la famille du platine (Os, Ir, Pt) et/ou en carbone vitreux, et des cathodes en un matériau usuel mais stable dans les conditions de l'électrolyse, on isole les ω-fluorosulfato-(méthyl-2 alcanones-3) formés, qui répondent à la formule V:

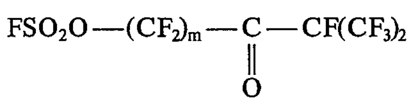
(V)

dans laquelle m a la signification indiquée ci-dessus, et

b) on décompose ces composés, en présence de quantités catalytiques d'un fluorure de métal alcalin MF (M désignant Li, K, Na, Rb, Cs) et en l'absence de solvants aprotiques polaires, de manière à obtenir les fluorures d'acides perfluoro-isopropyl-céto-carboxyliques de formule I.